Europäisches Patentamt

European Patent Office

Office européen des brevets

(11) Numéro de publication: **0 462 003 B1**

# FASCICULE DE BREVET EUROPEEN

(45) Date de publication de fascicule du brevet: **02.11.94**   (51) Int. Cl.5: **A61K 9/50**, A61K 9/52

(21) Numéro de dépôt: **91401561.5**

(22) Date de dépôt: **12.06.91**

Le dossier contient des informations techniques présentées postérieurement au dépôt de la demande et ne figurant pas dans le présent fascicule.

(54) **Microcapsule de liquide huileux.**

(30) Priorité: **13.06.90 FR 9007334**

(43) Date de publication de la demande:
**18.12.91 Bulletin 91/51**

(45) Mention de la délivrance du brevet:
**02.11.94 Bulletin 94/44**

(84) Etats contractants désignés:
**AT BE CH DE DK ES FR GB GR IT LI LU NL SE**

(56) Documents cités:
**EP-A- 0 225 303
EP-A- 0 336 662
WO-A-81/02976
GB-A- 2 223 943**

(73) Titulaire: **Université de Liège
7, Place du XX Août
B-4000 Liège (BE)**

(72) Inventeur: **Zgoulli, Slim
19 Av. Maréchal Juin
B-5800 Gembloux (BE)**
Inventeur: **Delfosse, Philippe
Rue Bois d'Avroy, 2B
B-4000 Liege (BE)**
Inventeur: **Thonart, Philippe
Rue Houlette 20
B-5850 Bovesse (BE)**
Inventeur: **Delacroix, Dominique
11 avenue des Ramiers
B-1950 Kraainem (BE)**

(74) Mandataire: **Van Malderen, Michel et al
p.a. Office van Malderen
85/043 Boulevard de la Sauvenière
B-4000 Liège (BE)**

Rank Xerox (UK) Business Services
(3.10/3.09/3.3.3)

## Description

La présente invention concerne des microcapsules, microparticules sphériques, constituées d'une enveloppe d'un matériau d'enrobage gastro-résistant constituant le réservoir d'un liquide huileux qu'elle contient.

La présente invention concerne également un produit fini sous forme de poudre formé de microcapsules selon l'invention libérant un principe actif de nature huileuse de manière retardée.

La présente invention concerne enfin un procédé de préparation de microcapsules et de produit fini sous forme de poudre selon l'invention.

En thérapeutique ou en prévention, on retrouve fréquemment le besoin de posséder une technique permettant le relargage d'un principe actif, après un séjour prolongé dans l'organisme.

En pharmacie galénique, les procédés les plus fréquents permettant d'obtenir ces effets sont :

1) La microencapsulation, par coacervation, avec des polymères gastro-résistants (H.P. MERKLE and SPEISER, J. Pharm. Sci. 62 : 1444-1448 (1973) ; L.A. LUZZI and R.J. GERRAUGHTY, J. Pharm. Sci. 53 : 429-431 (1964) ; J.W. BEYGER and J.G. NAIRN J. Pharm. Sci. 75, 6 : 573-578 (1986). Ces méthodes sont d'une mise en oeuvre délicate à l'échelle industrielle et font en outre souvent appel à des solvants organiques qui sont cause de résidus toxiques ou à des quantités de produits à mettre en oeuvre, qui rendent le produit fini économiquement inintéressant.

2) D'autre part, il existe un autre procédé, également très répandu, qui permet d'obtenir des formulations à libération retardée. Cette méthode n'est cependant applicable que sur des principes actifs se présentant sous une forme solide (poudre, granulés ou comprimés) et elle consiste en un enrobage (en lit d'air fluidisé ou en turbine) par projection d'une solution de polymère entérique sur des noyaux en suspension dans un lit d'air chaud (H.C. CALDWELL and E. ROSEN J. Pharm. Sci. 53 : 1387-1391 (1964) ; M.J. ROBINSON, G.M. GRASS and R.J. LANTZ J. Pharma. Sci. 57 : 1983-1988 (1968); K. LEHMANN, D. DREHER, Pharm. Ind. 34 : 894-899 (1972); W. ROTHE, G. GROPPENBÄCHER Pharm. Ind. 34 (11a) : 892-894 (1972)).

Cette technique n'est cependant pas applicable lorsqu'il s'agit d'encapsuler directement un principe actif sous forme liquide.

Dans l'industrie alimentaire, la technique d'encapsulation d'huiles alimentaires, d'arômes ou de colorants par des polymères non gastro-résistants tels que de la gomme arabique, la maltodextrine, la gélatine, est fort répandue. Ces applications sont réalisées, dans la majorité des cas, par le procédé d'atomisation ou "spray-drying" (ANANDARMAN, E.B. WILLIAM and G. REINECCIUS Perf. Flav. 8 : 49-56 (1983); K. IWAMI, M. HATTORI, S. NAKATANI and F. IBUKI, Agric. Biol. Chem., 51(12) : 3301-3307 (1987)).

Cette technique de préparation de microcapsules par séchage par atomisation n'a jamais été proposée et appliquée avec des polymères gastro-résistants en particulier tels que le CAP ou l'Eudragit$^R$ compte tenu de réelles difficultés de mise en oeuvre. Ces polymères ont en effet tendance à se présenter sous forme de filament et à bloquer la turbine de l'atomiseur, compte tenu de leur trop forte viscosité.

Le brevet EP 225 303 décrit des microcapsules d'huile qui sont préparées en émulsion aqueuse dans une solution de polymères gastro-résistants par la technique classique de coacervation ou par "spray-coating". La technique de "spray-coating" est une technique de vaporisation du polymère sur une poudre en lit fluidisé. La technique décrite dans ce brevet concerne des comprimés, des gélules ou capsules pouvant contenir de l'huile préparées en deux temps, le revêtement par un polymère gastro-résistant n'intervenant qu'après la préparation de la gélule ou capsule proprement dite.

Le brevet GB 2.223.943 décrit lui aussi des microcapsules. Il s'agit là encore de gélules, notamment de gélatine, remplies d'huile qui ne sont qu'après recouvertes d'un polymère gastro-résistant.

Le brevet EP 336 662 décrit la préparation de microcapsules contenant de l'huile préparées dans un premier temps à partir d'une émulsion d'huile dans l'eau (huile/alginate) qui est pulvérisée à froid dans une solution de CaCl$_2$, ce qui provoque la précipitation du polymère autour des gouttelettes d'huile. Ces microcapsules sont ensuite suspendues dans une solution du polymère gastro-résistant qui est précipité autour des microcapsules par ajout d'acide. Il s'agit donc là encore d'une technique d'encapsulation par coacervation chimique.

Le but de la présente invention est d'obtenir de manière originale une poudre gastro-résistante constituée de microcapsules comportant un liquide huileux.

## Eléments caractéristiques de l'invention

La présente invention concerne des microcapsules constituées par un enveloppe solide consistant en une couche de matériaux d'enrobage comportant au moins un polymère gastro-résistant en mélange avec un ou des polymère(s) d'enrobage non gastro-résistant(s) à propriété émulsifiante, ladite enveloppe solide contenant un liquide huileux.

Avantageusement, le polymère gastro-résistant est choisi parmi le groupe constitué par de l'acétyle phtalate de cellulose (CAP), du phtalate d'hydroxypropylméthyl cellulose, de l'acétyle phtalate de poiyvinyl, un copolymère d'acide méthacrylique et d'acide acrylique et/ou des protéines telles que les gliadines.

Selon l'invention, le matériau d'enrobage comporte à titre de polymère(s) non gastro-résistant(s) à propriétés émulsifiantes des polyinères choisis parmi le groupe constitué par des hydrocolloïdes polysaccharidiques tels que les gommes, notamment la gomme arabique, gomme du guar, gomme de Karaya, ou gomme de Caroube, des maltodextrines et/ou un mélange de ceux-ci.

Selon une forme d'exécution préférée de l'invention, le matériau d'enrobage comporte un mélange d'acétyle phtalate de cellulose, de gomme arabique et de maltodextrines.

En outre, le matériau d'enrobage comporte de préférence un agent plastifiant tel que de la triacétine, le propylène glycol, ou le cétanol, à une concentration variant de préférence entre 10 et 30% du poids du matériau d'enrobage.

Selon une autre forme d'exécution préférée de l'invention, le matériau d'enrobage est constitué d'un mélange d'acétyle phtalate de cellulose, de maltodextrine, de gomme arabique et d'un plastifiant choisi parmi la triacétine ou le propylène glycol.

Le liquide huileux des microcapsules consiste en une huile d'origine végétale ou animale, riche en acides gras polyinsaturés, notamment alpha-linolénique et linoléique libres ou sous forme estérifiée en triglycérides.

Selon une forme d'exécution préférée de l'invention, le liquide huileux comporte un mélange d'acides gras polyinsaturés dérivant des acides gras essentiels alpha-linolénique de la famille n-3 et alpha-linoléique de la famille n-6 ou de triglycérides enrichis en ces acides.

En outre, le liquide huileux contient de préférence un principe actif liposoluble.

Préférentiellement, les microcapsules selon l'invention ont une taille comprise entre 25 et 100μm.

Un autre aspect de l'invention concerne un produit fini sous forme de poudre constitué par microcapsules selon l'invention.

Un dernier aspect de l'invention concerne un procédé de préparation des microcapsules selon l'invention dans lequel on effectue un séchage par atomisation (spray-drying) d'une émulsion du type huile dans l'eau obtenue à partir du liquide huileux et d'une solution aqueuse de polymère(s) du matériau d'enrobage comportant au moins un polymère d'enrobage gastro-résistant en mélange avec au moins un agent émulsifiant, de préférence un polymère d'enrobage non gastro-résistant.

Selon l'invention, on réalise l'émulsification avec en outre un agent émulsifiant tensioactif non-ionique tel que le Tween 80R.

Avantageusement, l'émulsion formée est composée de fines gouttelettes de taille comprise entre 2 et 10 μm.

Selon une forme d'exécution préférée de l'invention, la solution aqueuse de polymères est une solution à 5 à 20% en poids de polymère(s), les polymères représentant 5 à 30% en poids de la matière sèche totale dans l'émulsion et le polymère gastro-résistant représentant 5 à 15% de la matière sèche de l'émulsion.

Dans la solution aqueuse de polymère(s), comportant de préférence 5,6% d'ammoniaque par rapport au poids du polymère, le polymère gastro-résistant est de préférence l'acétyle phtalate de cellulose solubilisé dans la solution aqueuse à un pH d'au moins 5,7 et est de préférence utilisé sous forme d'une solution aqueuse à 10% (p/p) dans une proportion variant entre 3 et 8% en matière sèche dans l'émulsion.

Dans le procédé selon l'invention, la gomme arabique et des maltodextrines sont utilisées en proportions égales de façon à atteindre une concentration totale en polymères de 15% en matière sèche dans l'émulsion.

De préférence, on réalise un premier mélange de polymères d'enrobage et un deuxième mélange de liquide huileux à encapsuler avec éventuellement un agent émulsifiant non-ionique puis on mélange lesdits premier et deuxième mélange entre eux et on ajoute finalement le cas échéant l'agent plastifiant.

De préférence, la quantité de liquide huileux représente environ 20 à 50%, en particulier 30%, de la matière sèche totale dans l'émulsifiant. Avantageusement, dans le procédé selon l'invention, l'agent émulsifiant est du Tween 80R dans une proportion de 0,1 à 1%, en particulier 0,5% (p/p) de matière sèche totale de l'émulsion.

Dans le procédé selon l'invention, on effectue le séchage par atomisation (spray-drying) à l'aide d'un atomiseur dont la température d'entrée est de 140°C.

Le but de la présente invention est d'obtenir de manière originale une poudre gastro-résistante constituée de microcapsules comportant un liquide huileux.

Pour ce faire, la présente invention fournit un procédé de préparation de microcapsules constituées d'une enveloppe solide d'un matériau d'enrobage comportant au moins un polymère gastro-résistant, l'enveloppe solide contenant un liquide huileux, procédé caractérisé en ce qu'on effectue un séchage par atomisation (encore appelé spray-drying), à l'aide d'un atomiseur, d'une émulsion du type huile-dans-l'eau obtenue à partir dudit liquide huileux et d'une solution aqueuse du matériau d'enrobage comportant le(s) polymère(s) gastro-résistant(s) en mélange avec au moins un agent émulsifiant.

Dans la présente demande, par matériau d'enrobage on entend désigner des polymères, y compris des gommes ou résines synthétiques ou des gommes ou résines naturelles, qui sont physiologiquement acceptables, voire des protéines.

La présente invention se base sur une encapsulation physique par atomisation (séchage) directe dans une tour de séchage d'une émulsion huile/eau formée par le principe actif et la solution aqueuse de polymère gastro-résistant en mélange avec d'autres polymères classiques de façon à récupérer directement en une étape une poudre sèche gastro-résistante, formée de microcapsules.

Le procédé selon l'invention implique donc la mise en oeuvre d'un dispositif de séchage par atomisation. Ces dispositifs sont bien connus de l'homme de l'art. Ils sont composés par exemple d'une haute tour au sommet de laquelle la phase liquide en l'occurrence l'émulsion, est finement dispersée par passage au travers d'un pulvérisateur à buse ou à disque rotatif. Les gouttelettes formées traversent un flux d'air établi à co ou à contre-courant et porté à une température contrôlée notamment de 140°C. Les microcapsules se solidifient et sont récupérées à la base de la chambre de pulvérisation. La pulvérisation est réalisée dans un courant d'air chaud. Le temps de contact des microcapsules avec l'air chaud est très bref et permet d'éviter au matériau enrobé d'atteindre une température supérieure à 40°C.

Ce procédé par atomisation ou nébulisation (spray-drying) offre l'intérêt d'une transposition très facile à l'échelle industrielle. La microencapsulation est réalisée en une seule étape et peut être menée en continu sur des lots importants.

Selon l'invention, on pourra utiliser avantageusement comme agent émulsifiant un polymère d'enrobage classique non gastro-résistant c'est-à-dire doté de propriétés filmogène et émulsifiante.

Aucune des microcapsules décrites avant l'invention ne possède une enveloppe constituée d'un polymère à propriétés gastro-résistantes en mélange avec d'autres polymères à propriétés émulsifiantes.

Selon l'invention, pour stabiliser l'émulsion, on pourra en outre, utiliser un agent émulsifiant tensio-actif non-ionique comme le Tween [80R].

Les polymères utilisés dans le procédé selon l'invention le sont sous forme de solution aqueuse qui permettent de réaliser une émulsion du type huile-dans-l'eau avec le liquide huileux. Aucun solvant organique n'est donc utilisé au cours du procédé de fabrication ce qui diminue considérablement les risques lors des manipulations et aussi la présence de résidus toxiques dans les produits finis.

La présentation du produit fini obtenu selon le procédé de l'invention est une poudre dont la taille des microcapsules la constituant est de 25 à 100 $\mu$. Cette présentation sous forme de poudre est particulièrement intéressante puisqu'elle peut être incorporée soit dans une formulation sous forme solide tels que des comprimés, des granulés, des dragées, soit sous une forme à disperser dans un liquide pour autant que celui-ci soit légèrement acide, par exemple des jus de fruits, de légumes, des limonades.

D'autres dispositifs d'atomisation utilisables selon l'invention sont décrits par exemple dans MARGARET M.L. "Spray drying of food flavors" Perfumer and Flavorist, 8, 49-56 ; YOUNG R.A. "Reviews the current for situation for spray drying encapsulation" J. Foods Londres Janvier (86) p. 31-33, Spray drying encapsulation todays view ; REINECCIUS G.A. et al. (1982) "Spray drying of Foods Flavors, J. theory of flavor retention" Perfumer and Flavorist, 7:4, 2-6.

Parmi les polymères gastro-résistants utiles selon l'invention, on peut citer plus particulièrement l'acétyl phtalate de cellulose (CAP), l'acétotriméllitate de cellulose (CAT), du phtalate d'hydroxypropylméthylcellulose (HP50 soluble à PH 5, HP55 soluble à PH 5,5), de l'acétyl phtalate de polyvinyl, un copolymère d'acide métacrylique, et d'acide acrylique tel que les produits de marque Eudragit[R], un latex préparé à base de CAP tel que les produits de marque Aquacoat[R], Aquateric[R] ou encore des protéines telles que les gliadines.

La liste qui précède n'est exhaustive mais illustre la gamme de polymères qui peuvent être utilisés pour produire des microcapsules selon l'invention.

Parmi les polymères non gastro-résistants utiles dotés de propriétés émulsifiantes selon l'invention, on peut citer des hydrocolloïdes polysaccharidiques telles les gommes, notamment la gomme arabique, la gomme guar, la gomme Karaya, la gomme de Caroube, les maltodextrines ou encore des mélanges de ceux-ci.

Là encore, cette liste n'est pas exhaustive mais illustre simplement la gamme de polymères qui peuvent être utilisés en mélange à un polymère gastro-résistant pour produire des microcapsules selon l'invention.

On peut citer plus particulièrement la gomme arabique, polysaccharide de poids moléculaire élevé dont le squelette principal est formé de D-galactose, fortement substitué par des groupes rhamnose, arabinose et acides glucuroniques salifiés (calcium, potassium, magnésium). Cet hydrocolloïde est soluble dans l'eau où il développe ses propriétés :

- texturantes et anticristallisante
- émulsifiante,
- filmogène et adhésive.

Son utilité au même titre que celle des autres gommes citées ci-dessus est double selon la présente invention :

- agent émulsifiant pour l'élaboration d'émulsion de type huile/eau,
- agent encapsulant, utilisé sur des huiles

De plus, il n'y a pas de limite aux doses journalières admissibles.

Les maltodextrines sont obtenues par l'hydrolyse de la molécule d'amidon, par un procédé identique à la préparation de sirops de glucose. L'amidon est un polymère de D-glucose, les chaînes linéaires sont obtenues par des liaisons 1-4, les ramifications par des liaisons 1-6. La molécule d'amidon, dégradée par l'hydrolyse à un stade plus ou moins avancé donnera un sirop de glucose qui sera caractérisé par la mesure du dextrose équivalent (D.E.).

Les maltodextrines DE 20 (sirop de glucose) sont des poudres blanches, solubles à froid dans l'eau, elles donnent des solutions peu visqueuses. Utilisées en proportion égale de la gomme arabique pour leurs propriétés filmogènes et encapsulantes, elles permettent de garder la viscosité de la solution de polymère à un niveau assez faible.

Dans un mode de réalisation, on peut utiliser une certaine quantité de plastifiants dans la solution de polymères. On peut citer

1) La triacétine (triacétate de glycérine) est un liquide de densité 1,15 et de point d'ébullition de 259°C. Elle est soluble dans 14 parties d'eau, dans l'alcool, le benzène, le chloroforme et l'éther.

2) Le propylèneglycol est un liquide limpide, incolore, inodore, de saveur légèrement sucrée, hygroscopique. Il est miscible à l'eau.

3) Le cétanol.

Ces plastifiants sont utilisés à des concentrations variant entre 10 et 30 % du poids des polymères d'enrobage, ils contribuent à la souplesse du film après séchage et facilitent l'atomisation de l'émulsion.

Dans un mode de réalisation particulier du procédé, le matériau comporte donc comme plastifiant la triacétine, le propylèneglycol ou le cétanol.

Le plastifiant peut être utilisé à une concentration variant entre 10 et 30 % du poids du matériau d'enrobage.

Préalable ment à la réalisation de l'émulsion, on effectue si nécessaire la solubilisation des polymères dans l'eau.

A titre de polymère gastro-résistant, on utilisera de préférence l'acétylphtalate de cellulose (CAP).

L'acétylphtalate de cellulose (CAP) est un dérivé de la cellulose, avec des groupements acétyl et acide phtalique qui y sont substitués, il est soluble dans l'eau, à partir des valeurs de pH supérieures à 5,7. Il est insoluble en milieu acide, ce qui lui confère ses propriétés de gastro-résistance.

Selon l'invention, il sera avantageusement utile sous la forme d'une solution aqueuse de 5 à 20 %, par exemple à 10 % (p/p), dans laquelle seront ajoutés 3 à 10 %, par exemple 5,6 % d'ammoniaque (p/p par rapport au polymère) pour le stabiliser. Tout l'ammoniaque est éliminé par évaporation lors de l'atomisation ; au cas où des résidus seraient présents dans le produit fini, on peut neutraliser ceux-ci par un lavage de la poudre à l'acide.

Lorsque le polymère gastro-résistant est l'acétylphtalate de cellulose, celui-ci est donc solubilisé dans une solution aqueuse a un pH d'au moins 5,7 par exemple de 5,7 à 10.

D'une manière générale, le polymère gastro-résistant, notamment l'acétylphtalate de cellulose pourra être utilisé sous forme d'une solution aqueuse de 5 à 20 %, en particulier à 10 % (p/p) dans une proportion variant entre 5 et 15 %, de préférence entre 3 et 8 % en matière sèche dans l'émulsion.

Le(s) polymère(s) non gastrorésistant(s) a propriété émulsifiante, notamment la gomme arabique et les maltodextrines seront avantageusement utilisés de façon à atteindre une concentration totale en polymère(s) de 5 à 30 %, en particulier 15 % en matière sèche dans l'émulsion.

La matière sèche totale dans l'émulsion correspond également au poids de la microcapsule obtenue.

Selon un mode de réalisation du procédé selon l'invention, on réalise un premier mélange du ou des polymères d'enrobage et un deuxième mélange du liquide huileux à encapsuler avec l'agent émulsifiant tensio-actif non-ionique le cas échéant, puis on mélange lesdits premier et deuxième mélanges entre eux et enfin on ajoute le cas échéant le plastifiant.

La quantité de liquide huileux engagée dans le procédé représentera en général environ 20 à 50 %, en particulier 30 % de la matière sèche totale dans l'émulsion.

L'agent émulsifiant tensio-actif non ionique utilisé dans le procédé sera de préférence du Tween 80[R], par exemple dans une proportion de 0,1 à 1%, en particulier 0,5 % (poids/poids) de l'émulsion.

A titre de liquide huileux, il est possible, par le procédé selon l'invention, d'encapsuler une grande gamme d'huiles d'origines diverses saturées ou insaturées, ou des mélanges d'acides gras ou de triglycérides.

En particulier, on pourra encapsuler les huiles alimentaires recommandées par les médecins et les nutrionnistes, notamment afin de prévenir les maladies cardiovasculaires, telles que l'huile d'arachide, l'huile de poissons riches en acides gras insaturés de la famille (n-3) (stéaridonique, eïcosapentaénoïque et docosahexaénoïque). On citera également l'huile de bourrache riche en acide gamma-linolénique et autres acides gras insaturés de la famille (n-6) qui permettent de pallier les déficiences de l'appareil enzymatique de synthèse des icosanoïdes.

Enfin, on citera des mélanges d'acides gras libres enrichis en acides gras intéressants, par un procédé d'hydrolyse enzymatique qui est décrit dans la demande de brevet FR 89 12980.

Dans un mode de réalisation préférentielle du procédé selon l'invention, les acides gras polyinsaturés contenus dans le liquide huileux dérivent des acides gras essentiels alpha-linoléniques et linoléïques. Il s'agit préférentiellement de l'acide gamma-linolénique, l'acide arachidonique, l'acide docesatétraénoïque, l'acide docosapentaénoïque, l'acide stéaridonique, l'acide eïcosapentaénoïque, et l'acide docosahexaénoïque.

Toutes les huiles contenant naturellement un ou plusieurs acides gras polyinsaturés essentiels sous forme libre ou sous forme de glycéride, conviennent donc particulièrement. Parmi celles-ci, on peut citer en particulier les huiles de poissons, tels que la sardine et la morue pour les acides gras polyinsaturés de la famille (n-3), et les huiles de bourrache DPO ou de pépins de cassis, pour les acides gras polyinsaturés essentiels de la famille (n-6).

Le produit liquide huileux encapsulé peut contenir n'importe quel principe actif que l'on désirerait introduire dans l'organisme au niveau de l'intestin, pour autant qu'il soit liposoluble. On peut citer par exemple les vitamines A, D, E et K, des colorants, des antibiotiques, mais aussi des peptides d'intérêt. On mentionne plus spécialement les principes actifs dont l'absorption intestinale est favorisée par leur association avec une huile.

On notera que, dans le cas où le liquide huileux ou le principe actif présente un goût ou une odeur désagréable, c'est le cas par exemple de l'huile de poissons, l'enrobage masque ceux-ci lors de la prise au niveau de la bouche, mais aussi l'enveloppe étant gastro-résistants, elle empêche sa libération au niveau de l'estomac et par là, des relents désagréables lors de la digestion.

L'encapsulation protège le principe actif de l'oxydation, ce qui permet un stockage du produit simplifié et prolongé. Pour augmenter cet effet, on peut mélanger le principe actif huileux avec de la vitamine E (alpha-tocophérol), c'est un anti-oxydant liposoluble. Comme on l'a vu, le procédé d'atomisation utilisé selon la présente invention, présente l'avantage de n'exposer le produit fini à une température élevée que pendant un temps très court (quelques secondes) ce qui diminue encore le risque d'oxydation du principe actif.

Les microcapsules obtenues par le procédé selon l'invention sont nouvelles et l'invention a donc également pour objet les microcapsules, à titre de produits nouveaux, caractérisées par une enveloppe solide consistant en une couche de matériaux d'enrobage comportant au moins un polymère gastro-résistant, ladite enveloppe solide contenant un liquide huileux ; et en particulier les microcapsules peuvent être caractérisées en ce qu'elles sont constituées, dans un mode de réalisation particulier, d'un polymère gastro-résistant en mélange avec un ou des polymère(s) d'enrobage non gastrorésistant(s) à propriété émulsifiante.

D'autres caractéristiques et avantages de la présente invention apparaîtront à la lumière de l'exemple détaillé qui va suivre.

EXEMPLE 1 : ATOMISATION D'UNE HUILE EN EMULSION DANS UNE SOLUTION DE CAP, MALTODEXTRINE, GOMME ARABIQUE ET TRIACETYL OU POLYPROPYLENEGLYCOLE

1) Matériel

| | |
|---|---|
| - gomme arabique : spray cleaned | (FEDERA) |
| - maltodextrine DE (20) | (VEL) |
| - acétyl phtalate de cellulose : CAP | (EASTMAN) |
| - huile de poisson | (ORTIS) |
| - Tween 80 | (FEDERA) |
| - Polypropylène glycol | (FEDERA) |
| - triacétine | (JANSSEN) |
| Mixer Ultra Turrax-T25 | (IKA VAN DER HEYDEN) |
| Atomiseur (Mobil Mixer Spray Dryer) | (NIRO ATOMISER) |

2) Méthodes

2.1.) Composition des émulsions

Les pourcentages s'entendent en rapport poids/poids des produits en émulsion, le reste étant constiuté d'eau distillée.

Tableau 1

| % en poids Exemples | CAP (%) | Gomme arabique (%) | MD20 (%) | plastifiant (%) | huile + 0,5 % tween80 (%) | MS (%) | eau (%) |
|---|---|---|---|---|---|---|---|
| n° 1 | 7 | 8 | - | - | 6,6 | 21,6 | 78,4 |
| n° 2 | 7 | 4 | 4 | - | 7,5 | 22,5 | 77,5 |
| n° 3 | 7 | 4 | 4 | 1,5(T) | 7,5 | 24,0 | 76 |
| n° 4 | 8 | 3,5 | 3,5 | 1,5(T) | 7,5 | 24,0 | 76 |
| n° 5 | 8 | 3,5 | 3,5 | 3,0(T) | 7,5 | 25,5 | 74,5 |
| n° 6 | 8 | 3,5 | 3,5 | - | 7,5 | 22,5 | 77,5 |
| n° 7 | 8 | 3,5 | 3,5 | 3,0(PG) | 8,0 | 26,0 | 74,0 |

MS : matière sèche dans l'émulsion MD20 : maltodextrine DE 20 (sirop de glucose) T : triacétine PG : propylèneglycol

2.2) Procédure

- Disperser le CAP dans une solution d'ammoniaque à 5 % en agitant pendant 45 minutes (solution I).
- Disperser la gomme arabique ; agitation magnétique pendant 30 minutes ; chauffer à 45°C (solution II).
- Ajouter la quantité de Maltodextrine DE 20-23 à la solution II, agitation pendant 20 minutes (solution III).
- Mélanger les solutions I et III, laisser agiter pendant 20 minutes.
- Ajouter le mélange huile-Tween 80 à la solution de polymères ; toujours avec agitation magnétique.
- Ajouter le plastifiant en agitant vigoureusement.

### 2.3) Formation de l'émulsion

Les émulsions sont réalisées par l'utilisation d'un Mixer Ultra-Turrax et sur une quantité d'échantillon variant entre 400 et 500 g.

Ainsi, pour l'exemple n°3, les quantités des différents constituants engagés dans le procédé peuvent être :

| Ingrédients | poids | en % |
|---|---|---|
| Gomme arabique | 16 g | 4,0 % |
| Maltodextrine DE20 | 16 g | 4,0 % |
| Acétylphtalate de cellulose : CAP | 28 g | 7,0 % |
| Huile de poisson + 0,5 % Tween 80 | 30 g | 7,5 % |
| Triacétine | 6 g | 1,5 % |
| Solution à 5 % $NH_4OH$ | 240 g | 60,0 % |
| Eau distillée | 64 g | 16,0 % |
| | 400 g | 100,0 % |

Afin de limiter l'échauffement du produit pendant l'émulsification, le récipient contenant le produit est plongé dans un bain de glace. La vitesse d'émulsification optimale est de 24.000 t/min, avec une tête de mixer pour dispersion fine (S25N-25F), pendant 20 minutes.

### 2.4) Evaluation de l'émulsion

a) mesure de l'indice turbidimétrique :
- une quantité de 50 $\mu$l d'émulsion est diluée dans 25 ml d'une solution de SDS (Sodium Dodecyl Sulfate) à 1 % pour éviter la coalescence pendant la mesure.
- mesure de la densité optique à 500 nm.

$$T = \frac{2,303 \ A}{L} \ (m^{-1})$$

A = densité optique
L = épaisseur de la cellule

b) observation au microscope

La méthode microscopique permet d'apprécier directement la finesse et la qualité d'une émulsion, par l'estimation de la taille des gouttelettes.

### 2.5) Séchage par atomisation

- Préparation de la chambre de séchage : après nettoyage et désinfection de la chambre, la machine est mise en marche environ 1/2 heure avant le début de l'atomisation. Ce laps de temps permet d'atteindre la température voulue à l'intérieur de la chambre et la bonne rotation de la turbine via le contrôle de la pression d'air.
- Température : à l'aide de sondes placées à l'entrée et à la sortie de la chambre, il est aisé de contrôler la variation de température entre ces deux points à tout instant.
- Avant l'atomisation, une fois que la chambre est sèche et qu'elle a atteint la température voulue, il est conseillé de commencer par l'injection d'eau distillée dans l'atomiseur. A ce moment, on observe une chute de la température de sortie due à l'évaporation de l'eau à l'intérieur de la chambre. Il est facile de contrôler la température de sortie en faisant varier le débit du liquide injecté.
- Une fois la température stabilisée, après environ 10 minutes on peut commencer l'injection de l'émulsion.

Les conditions d'atomisation sont les suivantes :

Débit du liquide : 28 ml/min

Pression d'air comprimé : 6 bars (± 30.000 t/min).
Température d'entrée : 140°C.
Température de sortie : 80-85°C.
- La poudre est récoltée dans un récipient fixé à la base du c yclone.

2.7) Dosage de l'huile

a) l'extraction de l'huile en surface :
L'hexane est utilisé pour extraire l'huile en surface à partir des microcapsules.
3 g de microcapsules sont pesés dans un tube à essai, 25 ml d'hexane PA sont ajoutés et on agite pendant 2 heures (agitateur rotatif).
Ensuite, on filtre sous vide, la poudre est lavée par 3 fois 8 ml d'hexane. On récupère l'éluat dans un ballon rodé, taré puis on évapore le solvant au rotavapor.
b) gastro-résistance :
Une prise d'essai de 3 g des microcapsules lavées à l'hexane est mise dans 20 ml d'HCl à 0,1 N sous agitation et dans un bain-marie à 37°C. Après deux heures d'incubation, une centrifugation est effectuée à 20.000 tours/minute pendant 20 minutes. Le surnageant est récupéré, deux fois 20 ml d'acétone y sont ajoutés. L'acétone ayant provoqué la précipitation de la gomme arabique et de la maltodextrine, on prélève la phase liquide après décantation. Les fractions récupérées sont extraites par deux fois 20 ml d'hexane dans une ampoule à décanter, la phase organique est séchée au rotavapor. Après évaporation, l'huile libérée est pesée.
Les motifs du choix de l'acétone et de l'hexane pour l'extraction de l'huile après incubation se resument en 3 points :
- L'acétone est miscible à la fois avec l'huile et avec l'eau, il n'y a qu'une seule phase.
- L'acétone précipite l'agent encapsulant (gomme arabique et maltodextrine) après l'extraction de l'huile, la séparation des phases liquidesolide peut se faire par décantation.
- L'hexane précipite le CAP et les plastifiants, il est miscible avec l'acétone et l'huile de poisson.
Les résultats (pourcentage d'huile relarguée dans le bain d'HCl 0,1 N après 2 heures à 37°C, par rapport au poids théorique total d'huile dans la poudre) sont présentés dans le tableau 6.

Tableau 6

| Exemples | % d'huile relarguée |
|----------|---------------------|
| 1 | 8,0 % |
| 2 | 3,6 % |
| 3 | 3,3 % |
| 4 | 1,0 % |
| 5 | 2,18 % |
| 6 | 3,2 % |
| 7 | 3,8 % |

3) Résultats

3.1) Etude de l'émulsion

La détermination de la taille des globules est une manière fiable d'évaluer l'émulsification et par conséquent, les caractéristiques et la stabilité des émulsions.
La méthode microscopique permet l'observation, le comptage, la mesure de la taille des globules et l'indice turbidimétrique est inversément proportionnel à la taille des gouttelettes.

a) Mesure de l'indice turbidimétrique en fonction de la durée d'émulsification

Les mesures sont présentées dans le tableau 2.

Tableau 2

| Durée d'émulsification (minutes) | Densité optique | Turbidité |
|---|---|---|
| 10 | 0,132 | 0,303 |
| 15 | 0,145 | 0,333 |
| 20 | 0,153 | 0,352 |
| 25 | 0,154 | 0,354 |

En théorie, l'augmentation du temps d'émulsification provoque la diminution de la taille des goutellettes. Le temps minimum nécessaire à l'obtention des globules d'une taille suffisamment petite est, dans nos conditions, de vingt minutes. La taille des gouttelettes après mesure au microscope se situe entre 2 et 10 $\mu$m.

b) Mesure de l'indice turbidimétrique en fonction de la vitesse du cisaillement du Mixer.

Les résultats sont présentés dans le tableau 3.

Tableau 3

| Vitesse t/min | densité optique | turbidité |
|---|---|---|
| 9400 | 0,125 | 0,287 |
| 13000 | 0,176 | 0,405 |
| 20000 | 0,276 | 0,626 |
| 24000 | 0,272 | 0,677 |

L'augmentation de l'énergie absorbée par la solution favorise la finesse et la stabilité de l'émulsion, jusqu'à ce que les gouttelettes atteignent une taille minimale, après quoi un apport d'energie supplémentaire peut conduire à une surémulsification.

c) Mesure de l'indice turbidimétrique en fontion de la concentration en Tween 80.

Les résultats sont présentés dans le tableau 4.

Tableau 4

| % en Tween/huile | densité optique | turbidité |
|---|---|---|
| 0,00 % | 0,240 | 0,552 |
| 0,05 % | 0,390 | 0,898 |
| 0,10 % | 0,444 | 1,022 |
| 0,20 % | 0,456 | 1,050 |

La fonction d'agent tensio-actif du Tween 80 renforce la cohésion du film en entourant les globules d'huile en émulsion. Si on utilise un agent encapsulant qui possède aussi des propriétés émulsifiantes, comme la gomme arabique, l'ajout de Tween 80 en très faible quantité renforce la stabilité de l'émulsion et donne au film une très grande résistance à l'étirement.

d) Conclusion

La finesse de l'émulsion dépend :
- de la vitesse de cisaillement de l'appareil utilisé pour le processus d'émulsification,
- de la durée de l'émulsification,
- de la concentration en agent tensio-actif,

3.2) Dosage de l'huile en surface

Les résultats sont présentés dans le tableau 5 :

Tableau 5

| Exemples | % huile en surface |
|---|---|
| 1 | 7 % |
| 2 | 6.5 % |
| 3 | 5.7 % |
| 4 | 4 % |
| 5 | 6.5 % |
| 6 | 7 % |
| 7 | 8 % |

On observe un taux faible d'huile en surface, ceci peut être expliqué de deux façons :
- des gouttelettes d'huile de petite taille favorisent la formation de microcapsules étanches,
- dans le domaine de température étudié, une température d'entrée de 140°C semble un bon compromis entre un bon rendement d'encapsulation, une rapidité de séchage suffisante et une bonne qualité de produit fini.

**Revendications**

**1.** Microcapsules constituées par une enveloppe solide consistant en une couche de matériau d'enrobage comportant au moins un polymère gastro-résistant en mélange avec un ou des polymère(s) d'enrobage non gastrorésistant(s) à propriété émulsifiante, ladite enveloppe solide contenant un liquide huileux.

**2.** Microcapsules selon la revendication 1 dans lesquelles le polymère gastro-résistant est choisi parmi le groupe constitué par de l'acétyle phtalate de cellulose (CAP), du phtalate d'hydroxypropylméthyl cellulose, de l'acétyle phtalate de polyvinyl, un copolymère d'acide méthacrylique et d'acide acrylique et/ou des protéines telles que les gliadines.

**3.** Microcapsules selon l'une des revendications 1 ou 2 dans lesquelles le matériau d'enrobage comporte à titre de polymère(s) non gastrorésistant(s) à propriétés émulsifiantes des polymères choisis parmi le groupe constitué par des hydrocolloïdes polysaccharidiques tels que les gommes, notamment la gomme arabique, gomme du guar, gomme de Karaya, ou gomme de Caroube, des maltodextrines et/ou un mélange de ceux-ci.

**4.** Microcapsules selon l'une quelconque des revendications précédentes dans lesquelles le matériau d'enrobage comporte un mélange d'acétyle phtalate de cellulose, de gomme arabique et de maltodextrines.

**5.** Microcapsules selon l'une quelconque des revendications précédentes dans lesquelles le matériau d'enrobage comporte un agent plastifiant tel que la triacétine, le propylène glycol, ou le cétanol.

**6.** Microcapsules selon l'une quelconque des revendications précédentes dans lesquelles le plastifiant est à une concentration variant entre 10 et 30% en poids du matériau d'enrobage.

**7.** Microcapsules selon l'une quelconque des revendications précédentes dans lesquelles le matériau d'enrobage est constitué d'un mélange d'acétyle phtalate de cellulose, de maltodextrine, de gomme

11

arabique et d'un plastifiant choisi parmi la triacétine ou le propylène glycol.

8. Microcapsules selon l'une quelconque des revendications précédentes dans lesquelles le liquide huileux consiste en une huile d'origine végétale ou animale, riche en acides gras polyinsaturés, notamment alpha-linolénique et linoléique libres ou sous forme estérifiée en triglycérides.

9. Microcapsules selon l'une quelconque des revendications précédentes dans lesquelles le liquide huileux comporte un mélange d'acides gras polyinsaturés dérivant des acides gras essentiels alpha-linolénique de la famille n-3 et alpha-linoléique de la famille n-6 ou de triglycérides enrichis en ces acides.

10. Microcapsules selon l'une quelconque des revendications précédentes dans lesquelles le liquide huileux contient un principe actif liposoluble.

11. Microcapsules selon l'une quelconque des revendications précédentes, de taille comprise entre 25 et 100 $\mu$m.

12. Produit fini sous forme de poudre constitué par des microcapsules selon l'une quelconque des revendications précédentes.

13. Procédé de préparation d'une microcapsule selon l'une quelconque des revendications précédentes, dans lequel on effectue un séchage par atomisation d'une émulsion du type huile dans l'eau obtenue à partir dudit liquide huileux et d'une solution aqueuse de polymère(s) du matériau d'enrobage comportant au moins un polymère d'enrobage gastro-résistant en mélange avec au moins un agent émulsifiant.

14. Procédé selon la revendication 13 dans lequel un agent émulsifiant est un polymère d'enrobage non gastrorésistant.

15. Procédé selon l'une quelconque des revendications 13 et 14, dans lequel on réalise l'émulsification avec en outre un agent émulsifiant tensioactif non-ionique.

16. Procédé selon l'une quelconque des revendications précédentes dans lequel l'émulsion formée est composée de fines gouttelettes de taille comprise entre 2 et 10 $\mu$m.

17. Procédé selon l'une quelconque des revendications précédentes dans lequel la solution aqueuse de polymère(s) est une solution à 5 à 20% en poids de polymère(s), les polymère(s) représentant 5 à 30% en poids de la matière sèche totale dans l'émulsion, et le polymère gastro-résistant représentant 5 à 15% de la matière sèche de l'émulsion.

18. Procédé selon l'une quelconque des revendications précédentes dans lequel dans la solution aqueuse de polymère(s), le polymère gastro-résistant est de l'acétyle phtalate de cellulose solubilisé dans la solution aqueuse à un pH d'au moins 5,7.

19. Procédé selon la revendication 18 dans lequel la solution aqueuse comporte 5,6% d'ammoniaque par rapport au poids du polymère.

20. Procédé selon la revendication 19 dans lequel l'acétyle phtalate de cellulose est utilisé sous forme d'une solution aqueuse à 10% (p/p) dans une proportion variant entre 3 et 8% en matière sèche dans l'émulsion.

21. Procédé selon l'une quelconque des revendications précédentes dans lequel la gomme arabique et des maltodextrines sont utilisées en proportions égales de façon à atteindre une concentration totale en polymères de 15% en matière sèche dans l'émulsion.

22. Procédé selon l'une quelconque des revendications précédentes dans lequel on réalise un premier mélange des polymères d'enrobage, et un deuxième mélange de liquide huileux à encapsuler avec éventuellement un agent émulsifiant non-ionique, puis on mélange lesdits premier et deuxième mélanges entre eux et enfin, on ajoute le cas échéant l'agent émulsifiant.

**23.** Procédé selon l'une quelconque des revendications précédentes dans lequel la quantité de liquide huileux représente environ 20 à 50%, en particulier 30%, de la matière sèche totale dans l'émulsion.

**24.** Procédé selon l'une quelconque des revendications précédentes dans lequel l'agent émulsifiant est un agent tensioactif non-ionique dans une proportion de 0,1 à 1%, en particulier 0,5%, (poids/poids) de la matière sèche totale de l'émulsion.

**25.** Procédé selon l'une quelconque des revendications précédentes dans lequel on effectue le séchage par atomisation à l'aide d'un atomiseur dont la température d'entrée est de 140°C.

**Claims**

**1.** Microcapsules made with a solid sheath consisting in a layer of a coating material comprising at least one gastro-resistant polymer mixed with one or various non gastro-resistant coating polymer(s) with emulsifying properties, the said solid sheath containing a liquid oil.

**2.** Microcapsules according to claim 1, wherein the gastro-resistant polymer is selected amongst the group of cellulose acetyl phthalate (CAP), cellulose hydroxypropylmethyl phthalate, polyvinyl acetyl phthalate, a copolymer of methacrylic acid and acrylic acid and/or proteines such as gliadines.

**3.** Microcapsules according to any of claims 1 or 2, wherein the coating material comprises, as non gastro-resistant polymer(s) with emulsifying properties, polymers seclected amongst the group of polysaccharide hydrocolloids such as gums, including arabic gum, guar gum, Karaya gum or carob gum, maltodextrins and/or mixtures thereof.

**4.** Microcapsules according to any of preceding claims, wherein the coating material comprises a mixture of cellulose acetyl phthalate, arabic gum and maltodextrins.

**5.** Microcapsules according to any of preceding claims, wherein the coating material comprises a plastifier such as triacetine, propylene glycol or cetanol.

**6.** Microcapsules according to any of preceding claims, wherein the plastifier is present in an amount between 10 and 30 wt.% on basis of the coating material.

**7.** Microcapsules according to any of preceding claims, wherein the coating material is made of a mixture of cellulose acetyl phthalate, maltodextrin, arabic gum and a plastifier selected amongst triacetine or propylene glycol.

**8.** Microcapsules according to any of preceding claims, wherein the oily liquid consists in a vegetal or animal oil, rich in polyinsaturated fatty acids, including free alpha-linolenic and linolenic ones or as an esterified form into triglycerides.

**9.** Microcapsules according to any of preceding claims, wherein the oily liquid comprises a mixture of polyinsaturated fatty acids derivated from alpha-linoleic essential fatty acids of family n-3 and alpha-linoleic ones of family n-6 or triglycerides enriched with those acids.

**10.** Microcapsules according to any of preceding claims, wherein the oily liquid contains a liposoluble active agent.

**11.** Microcapsules according to any of preceding claims, of a size between 25 and 100 $\mu$m.

**12.** Finished product as a powder formed with microcapsules according to any of preceding claims.

**13.** Process of preparation of a microcapsule according to any of preceding claims, wherein a drying step is carried out by atomisation of an emulsion of an oil-in-water type obtained from the said oily liquid and an aqueous solution of polymer(s) of the said coating material comprising at least one gastro-resistant coating polymer mixed with at least one emulsifier.

13

**14.** Process according to claim 13, wherein one emulsifier is a non gastro-resistant coating polymer.

**15.** Process according to any of claims 13 and 14, wherein the emulsification is carried out with, in addition, a non ionic surface-active emulsifier.

**16.** Process according to any of preceding claims, wherein the emulsion is made of fine droplets of a size comprised between 2 and 10 $\mu$m.

**17.** Process according to any of preceding claims, wherein the aqueous solution of polymer(s) is a solution with 5 to 20 wt.% of polymer(s), the polymer(s) representing 5 to 30 wt.% of the total dry material in the emulsion, and the gastro-resistant polymer representing 5 to 15% of the dry material of the emulsion.

**18.** Process according to any of preceding claims, wherein in the aqueous solution of polymer(s), the gastro-resistant polymer is cellulose acetyl phthalate solubilized in the aqueous solution at pH of at least 5,7.

**19.** Process according to claim 18, wherein the aqueous solution comprises 5,6% ammonia on basis of the polymer weight.

**20.** Process according to claim 19, wherein the cellulose acetyl phthalate is used as an 10% (w/w) aqueous solution in an amount between 3 and 8% dry material in the emulsion.

**21.** Process according to any of preceding claims, wherein the arabic gum and maltodextrins are used in equal amounts so as to reach a total concentration of polymers of 15% dry material in the emulsion.

**22.** Process according to any of preceding claims, wherein a first mixture of the coating polymers is carried out and then a second mixture of the oily liquid to be encapsulated with optionally a non ionic emulsifier, and then the said first and second mixtures are mixed to each other and finally the emulsifier is added the case being.

**23.** Process according to any of preceding claims, wherein the amount of oily liquid represents 20 to 50%, particularly 30%, of the total dry material in the emulsion.

**24.** Process according to any of preceding claims, wherein the emulsifier is a non ionic surfactant in an amount of 0.1 to 1%, particularly 0.5% (w/w) of the total dry material of the emulsion.

**25.** Process according to any of preceding claims, wherein the drying by atomization is made with an atomizer, the input temperature of which is 140°C.

**Patentansprüche**

**1.** Mikrokapseln, mit einer festen Hülle, die aus einer Schicht eines Umhüllungsmaterials besteht, das mindestens ein gastroresistentes Polymer, vermischt mit einem oder mehreren nicht-gastroresistenten Umhüllungspolymeren mit emulgierender Eigenschaft umfaßt, wobei die feste Hülle eine ölige Flüssigkeit enthält.

**2.** Mikrokapseln gemäß Anspruch 1, bei denen das gastroresistente Polymer aus der Gruppe ausgewählt ist, die von celluloseacetylphthalat (CAP), Hydroxypropylmethylcellulosephthalat, Polyvinylacetylphthalat, einem Copolymer aus Methacrylsäure und Acrylsäure, und/oder Proteinen, wie beispielsweise Gliadinen gebildet wird.

**3.** Mikrokapseln gemäß einem der Ansprüche 1 oder 2, bei denen das Umhüllungsmaterial als nicht-gastroresistente Polymere mit emulgierenden Eigenschaften Polymere umfaßt, die aus der Gruppe ausgewählt sind, die von Polysaccharidhydrokolloiden, wie beispielsweise Gummis, insbesondere Gummi arabicum, Guargummi, Karayagummi oder Caroubegummi, Maltodextrinen und/oder einem Gemisch davon gebildet wird.

14

EP 0 462 003 B1

4. Mikrokapseln gemäß irgendeinem der vorhergehenden Ansprüche, bei denen das Umhüllungsmaterial ein Gemisch aus Celluloseacetylphthalat, Gummi arabicum und Maltodextrinen umfaßt.

5. Mikrokapseln gemäß irgendeinem der vorhergehenden Ansprüche, bei denen das Umhüllungsmaterial ein Plastifizierungsmittel, wie beispielsweise Triacetin, Propylenglykol oder Cetanol umfaßt.

6. Mikrokapseln gemäß irgendeinem der vorhergehenden Ansprüche, bei denen das Plastifizierungsmittel eine zwischen 10 und 30 Gewichtsprozent des Umhüllungsmaterials variierende Konzentration hat.

7. Mikrokapseln gemäß irgendeinem der vorhergehenden Ansprüche, bei denen das Umhüllungsmaterial aus einem Gemisch von Celluloseacetylphthalat, Maltodextrin, Gummi arabicum, sowie Triacetin oder Propylenglykol als Plastifizierungsmittel besteht.

8. Mikrokapseln gemäß irgendeinem der vorhergehenden Ansprüche, bei denen die ölige Flüssigkeit aus einem Öl pflanzlichen oder tierischen Ursprungs besteht, das reich an Polyenfettsäuren, insbesondere Alpha-Linolen- Fettsäure und Linol-Fettsäure in freier oder zu Triglyzeriden esterifizierter Form ist.

9. Mikrokapseln gemäß irgendeinem der vorhergehenden Ansprüche, bei denen die ölige Flüssigkeit ein Gemisch von Polyenfettsäuren umfaßt, die von den essentiellen Alpha-Linolen-Fettsäuren der Familie n-3 und den essentiellen Alpha-Linol-Fettsäuren der Familie n-6, oder von mit diesen Säuren angereicherten Triglyzeriden abgeleitet sind.

10. Mikrokapseln gemäß irgendeinem der vorhergehenden Ansprüche, bei denen die ölige Flüssigkeit einen fettlöslichen Wirkstoff enthält.

11. Mikrokapseln gemäß irgendeinem der vorhergehenden Ansprüche, von einer Größe zwischen 25 und 100 $\mu$m.

12. Pulverförmiges Endprodukt, das aus Mikrokapseln gemäß irgendeinem der vorhergehenden Ansprüche besteht.

13. Verfahren zur Herstellung einer Mikrokapsel gemäß irgendeinem der vorhergehenden Ansprüche, bei dem eine Trocknung ausgeführt wird durch Zerstäubung einer Öl-in-Wasser-Emulsion, die aus der öligen Flüssigkeit und einer wässerigen Polymerlösung des Umhüllungsmaterials erhalten wurde, das mindestens ein gastroresistentes Umhüllungspolymer, vermischt mit mindestens einem Emulgiermittel umfaßt.

14. Verfahren gemäß Anspruch 13, bei dem ein Emulgiermittel ein nicht-gastroresistentes Umhüllungspolymer ist.

15. Verfahren gemäß irgendeinem der Ansprüche 13 und 14, bei dem die Emulgierung außerdem mit einem nicht-ionischen grenzflächenaktiven Emulgiermittel ausgeführt wird.

16. Verfahren gemäß irgendeinem der vorhergehenden Ansprüche, bei dem die gebildete Emulsion aus feinen Tröpfchen mit einer Größe zwischen 2 und 10 $\mu$m besteht.

17. Verfahren gemäß irgendeinem der vorhergehenden Ansprüche, bei dem die wässerige Polymerlösung eine Lösung mit 5 bis 20 Gewichtsprozent Polymer(en) ist, wobei die Polymere 5 bis 30 Gewichtsprozent des gesamten Trockenmaterials in der Emulsion repräsentieren, und das gastroresistente Polymer 5 bis 15% des Trockenmaterials der Emulsion repräsentiert.

18. Verfahren gemäß irgendeinem der vorhergehenden Ansprüche, bei dem in der wässerigen Polymerlösung das gastroresistente Polymer Celluloseacetylphthalat ist, das in der wässerigen Lösung bei einem pH von mindestens 5,7 löslich gemacht ist.

19. Verfahren gemäß Anspruch 18, bei dem die wässerige Lösung 5,6% Ammoniak bezüglich des Gewichts des Polymers umfaßt.

15

**20.** Verfahren gemäß Anspruch 19, bei dem das Celluloseacetylphthalat in Form einer 10%igen wässerigen Lösung (nach Gewicht) bei einem Trockenmaterial-Anteil von 3 bis 8% in der Emulsion verwendet wird.

**21.** Verfahren gemäß irgendeinem der vorhergehenden Ansprüche, bei dem das Gummi arabicum und Maltodextrine bei gleichen Anteilen so verwendet werden, daß eine gesamte Polymer-Konzentration von 15% Trockenmaterial in der Emulsion erreicht wird.

**22.** Verfahren gemäß irgendeinem der vorhergehenden Ansprüche, bei dem ein erstes Gemisch der Umhüllungspolymere, und ein zweites Gemisch der einzukapselnden öligen Flüssigkeit mit eventuell einem nicht-ionischen Emulgiermittel verwirklicht wird, danach das erste und das zweite Gemisch miteinander gemischt werden, und schließlich gegebenenfalls das Emulgiermittel zugegeben wird.

**23.** Verfahren gemäß irgendeinem der vorhergehenden Ansprüche, bei dem die Menge der öligen Flüssigkeit ungefähr 20 bis 50%, vorzugsweise 30%, des gesamten Trockenmaterials in der Emulsion repräsentiert.

**24.** Verfahren gemäß irgendeinem der vorhergehenden Ansprüche, bei dem das Emulgiermittel ein nicht-ionisches grenzflächenaktives Mittel mit einem Anteil von 0,1 bis 1%, vorzugsweise 0,5%, (nach Gewicht) an dem gesamten Trockenmaterial in der Emulsion ist.

**25.** Verfahren gemäß irgendeinem der vorhergehenden Ansprüche, bei dem die Trocknung durch Zerstäubung mittels eines Zerstäubers, dessen Eingangstemperatur 140 °C beträgt, ausgeführt wird.